# EUROPEAN PATENT SPECIFICATION

(11) **EP 4 482 545 B1**
(45) Date of publication and mention of the grant of the patent: **17.12.2025**
(21) Application number: 23706870.5
(22) Date of filing: 16.02.2023
(51) Int. Cl.: A61L 27/36, A61L 27/44, A61L 27/60, G01N 33/50

(54) **METHOD FOR PRODUCING TISSUE CONSTRUCTS**
VERFAHREN ZUR PRODUKTION VON GEWEBEKONSTRUKTEN
PROCÉDÉ DE PRODUCTION DE CONSTRUCTIONS TISSULAIRES

(30) Priority: 22.02.2022 EP 22158003
(43) Date of publication of application: 01.01.2025
(73) Proprietor: MIMIX BIOTHERAPEUTICS SA, 2503 Biel / Bienne (CH)
(72) Inventor: NESIC, Dobrila, 1207 Genève (CH); DI MARZIO, Nicola, 7270 Davos Platz (CH); THURNER, Marc, 2075 Wavre (CH); SERRA, Tiziano, 7270 Davos Platz (CH)
(74) Representative: P&TS SA (AG, Ltd.)
(86) International application number: PCT/IB2023/051401
(87) International publication number: WO 2023/161769

(56) References cited:
- WO-A2-2006/097707
- US-A1- 2013 085 079
- US-A1- 2014 274 802
- US-B2- 9 688 962

## Description

### Technical domain

The present invention relates to methods for producing a human or an animal tissue construct, for example a tissue graft.

### Related art

The replacement of damaged tissue by suitable transplants for patients suffering from burns, chronic diseases and other injuries or disease continues to be an urgent and not sufficiently addressed need in our time. To avoid complications and to ensure acceptance of a transplant by the patient's immune system, autografts, i.e. grafts of the patient's own tissue, are the preferred option for reconstructive surgery. Especially in life threatening emergency situation (e.g severe burns) the accessibility to donor tissue is often limited if not impossible.

The amount of tissue sample which can be obtained from an injured, diseased of burned patient, is however often limited, in particular if large parts of the respective tissue are defective, as is often the case with second- or third-degree burn patients.

To avoid the patient having to rely on allografts obtained from other donor tissue, different approaches have been developed to produce and enlarge a patient's own tissue to provide tissue grafts for surgical transplantation.

The most commonly used approach for production of skin grafts today is the meshing of split skin grafts, used in about 80 % of all skin grafting surgeries performed. In this method, a healthy skin tissue sample is surgically harvested from a patient and multiple staggered incisions are made at regular intervals in the tissue. The graft is then stretched and transplanted onto the patient in its expanded state. The higher the number of incisions, the further a graft can be stretched. A meshed split skin graft can theoretically expand up to 9-fold. However, a high number of incisions slows down the healing process and affects esthetical and functional outcome. Meshed split skin grafts are therefore more usually only expanded about 4 to 5-fold.

Tissue engineering provides a suitable alternative to conventional autologous grafting methods. The discipline is rapidly evolving and has delivered promising approaches for tissue grafting.

WO2021100047 discloses a method for producing skin grafts in a closed, automated system. The method comprises the isolation and separation of fibroblasts and keratinocytes from a skin biopsy sample, expanding the separated cells and subsequently transferring processed keratinocytes and fibroblasts in a device for formation of the tissue. Even though the process is automated, the separation, expansion and processing of cells is time consuming and the preparation of skin graft can take up to several weeks.

This timeframe is unsuitable for patients who urgently require skin grafts, for example as a result of accidents or due to other emergency situations. Moreover, the tissue needs to be produced in a GMP laboratory, which is decentralized from the point of treatment. The graft production therefore implies significant logistic and regulatory complexity.

In recent years, sound-induced morphogenesis (SIM) was found to be a powerful technology to generate biological structures and architectures. The application of sound energy to biological material such as cells, organoids or tissue fragments in a static acoustic wave field conducts the biological material into forming patterns defined by the standing waves.

The technology is versatile and perfectly suited for generating organized cell and particulate entity assemblies and for tissue engineering constructs.

US9688962 discloses a method of inducing spatial organization of cells in an in vitro culture system using ultrasound technology. This document teaches the use of this method for three-dimensional in vitro extracellular matrix remodelling and neovascularisation using a polymerised biological support structure as a scaffold.

WO2006097707 concerns a method for modulating the tensile strength and/or elastic strength of suspension of living cells by ultrasound stimulation, as well as a composition comprising such living cells having modulated tensile strength and/or elastic strength. The invention is directed to skin equivalents.

US20140274802 concerns an engineered, three-dimensional liver tissue construct and a method or producing the construct using bio-inks. The invention does not use SIM for producing the three-dimensional liver tissue constructs.

This invention aims to provide of a method to produce tissue constructs, which may for example be used as tissue grafts, such as skin grafts, that overcomes the shortcomings and limitations of the state of the art.

### Short disclosure of the invention

It is an aim of the invention to produce a tissue construct, which closely resembles natural human or animal tissue.

It is a further aim of the invention to increase the speed of production of tissue constructs, such as tissue grafts. A rapid fabrication of a graft makes the method suitable for provision of required tissue grafts in emergency situations. Preferably the invention should provide for the production of a point-of-care tissue graft. The graft should be safe for the patient and effectively support internal or external wound healing.

It is a further aim of the invention to increase the degree of enlargement of a tissue sample collected for the production of the tissue graft while aiming at reducing the donor site dimension. The expansion should be increased, such as to allow for the production of tissue grafts with a significantly larger surface area than conventionally used methods. The large tissue graft should be ready for application in a relatively short amount of time.

It is another aim of the invention to produce a tissue graft which is compatible with a recipient's immune system. The risk of rejection of a tissue graft is thereby minimized.

It is a further aim of this invention to provide tissue constructs for diagnostic analysis, which can be rapidly produced in a short amount of time and only require a small amount of patient tissue sample.

According to the invention, one or more of these aims are attained by the object of the attached claims, and especially by its independent claims. Further optional embodiments are provided in the dependent claims.

In particular, one or more aims are attained by a method for producing a human or animal tissue construct, comprising providing a tissue sample or a plurality of microtissues.

If a tissue sample is provided, cells contained in the tissue sample should be mobile in the sample. Suitable tissue samples are for example blood tissue, a bone marrow sample, or adipose tissue.

The term microtissues as used herein includes micrografts, organoids, spheroids, microvascular fragments, and cell aggregates.

Microtissues provide a more accurate representation of animal or human tissue than a mere suspension of one or more types of cells in tissue culture. The cell density and distribution in microtissues typically matches that of a natural tissue. In this environment biological processes and functions observed in natural human or animal tissue are induced. This environment arises primarily due to the inherent cell density and the degree of internal cell-to cell interactions within microtissues. It is a result of cell distribution and cell density enhancement.

Spheroids are three-dimensional spherical cellular agglomerates having a low degree of complexity. Spheroids may comprise one differentiated cell type, or they may comprise a variety of differentiated cell types. Spheroids may contain proliferating cells and/or non-proliferating cells. The cells can be immortalized cells and/or primary cells.

Differently from spheroids, organoids arise from tissue-specific stem cells, show a high degree of complexity and are usually designed to mimic a particular organ or function of an organ. They may be seen as a simplified mini-organs.

The size of organoids and spheroids typically varies, with typical transversal dimensions ranging from 50 µm to 200 µm.

Micrografts are particularly useful for generating tissue grafts for transplantation. Micrografts may be prepared from a collected sample tissue, which is segregated into micrografts.

Depending on the desired nature of a tissue construct, the collected tissue sample may be obtained from specific donor tissues. The collected tissue sample may be a skin sample. The sample may also be an adipose tissue sample, or a bone marrow sample. The sample may comprise microvascular fragments for lymphatic tissue engineering.

Segregation of a collected sample tissue, in particular a solid sample tissue, into micrografts is preferably carried out using a disaggregation method, which may be a mechanical, a chemical disaggregation method, or a combination of both. Any segregation or disaggregation method, which does not impact significantly on the cells' ability to proliferate and differentiate is suitable for the method described in invention.

The term micrografts as used herein means portions of tissues or multicellular structures having a maximum transversal dimension of no more than 2 mm, no more than 1 mm, no more than 500 µm, or no more than 200 µm, preferably of no more than 100 µm, for example 80 µm ± 20 µm. Preferably the micrografts are autologous micrografts retrieved from a biological tissue sample of a patient for whom the tissue construct is produced.

The invention is not particularly limited to the type of microtissue provided for the generation of the tissue construct. The purpose and the intended use of the construct will define the suitable sample tissue to be provided.

The method further comprises the provision of a collected blood sample. The blood sample may be a whole blood sample, preferably venous blood. The blood sample may be freshly collected. The blood sample may also be a previously collected and stored blood sample.

The blood sample may also be a processed blood sample.

The blood sample provided for the tissue construct is preferably an autologous blood sample, in particular if the tissue construct is intended to be used as a tissue graft for auto-transplantation. In this case both, the blood sample as well as the micrografts should be autologous, i.e. collected from the intended recipient of the tissue graft.

A processed blood sample may for example be obtained through centrifugation of whole blood, centrifugation of clotted blood, or centrifugation of blood comprising anti-coagulants. The processed sample may be blood plasma, blood serum, or other fragmentations of blood. The processed blood sample comprises all necessary coagulation promoting components for coagulation of the processed blood sample.

To induce, expedite or promote coagulation in a whole blood sample or in a processed blood sample, the samples may be supplemented with coagulation promoting components, such as fibrin or fibrinogen, calcium ions, platelets and/or clotting factors known in the art. Certain vitamins, such as vitamin K, or analytes known in the art to expedite the coagulation process, may also be provided as coagulation promoting components.

Coagulation promoting components are at least fibrin, or fibrinogen. If fibrinogen is provided in addition to or instead of fibrin, thrombin needs to be added to process the fibrinogen into fibrin. If the sample does not contain platelets, such as a serum sample, it needs to be supplemented with platelets. A sample, in particular a serum sample, may be supplemented with clotting factors or with other coagulation promoting components.

Clotting factors include for example pro-enzymes or enzymes, such a proteases of the clotting cascade, prothrombin and thrombin.

Coagulation promoting components, such as clotting factors, are preferably provided at concentrations, which are known to induce the coagulation cascade and cause coagulation of blood. Concentrations of individual components or a combination of components may be increased to promote the coagulation process.

Preferably the concentration of any clotting factor or other coagulation promoting component is provided in the range of ± 40%, or ± 20% of its average concentration in a whole blood sample exhibiting normal coagulation activity.

Since blood serum does not coagulate, a serum sample needs to be supplemented with the necessary coagulation promoting components.

Coagulation promoting components may be added to whole blood samples or to processed blood samples to expedite the coagulation process. The addition of coagulation promoting components may also be added to alter the consistency of the generated coagulated sample, for example to generate a coagulated sample with a denser fibrin scaffold. By adding or re-introducing coagulation promoting components, different types of blood-based matrices can be obtained for the tissue construct.

Alternatively, it may be desirable to decelerate the natural coagulation process of the blood sample or processed blood sample for the production of the tissue construct. To this end an anti-coagulant may be added to the sample, such as heparin, warfarin or ethylenediaminetetraacetic acid ("EDTA"). The anti-coagulant must however be provided at a concentration which merely delays blood coagulation, but does not prevent the sample from coagulating entirely.

It is also possible to use a homologous, also called allogenic, blood sample derived from a blood donor or from a blood bank for the method described herein.

If the micrograft is produced for diagnostic purposes, autologous or allogenic blood may be used. Even though heterologous blood samples might also be used, this is not suited for the production of tissue grafts. Heterologous blood is also not preferred for constructs used in diagnostics. For experimental purposes autologous, homologous, or heterologous blood may be used.

The plurality of microtissues or the tissue sample, which preferably comprises freely migrating cells, are suspended in a volume of collected blood and positioned in a receptacle having preferably a flat base. At least a portion of the base of the receptacle should be flat or substantially flat. Optionally, the tissue sample or microtissues may be washed in a suitable buffer, for example a phosphate-buffered saline, prior to being suspended in the blood.

As an example, 8'000 to 12'000 micrografts having an average diameter of about 80 µm may be suspended in 4 ml of collected blood.

The receptacle is preferably dimensioned to define the surface area of the produced tissue construct. The dimensions of the receptacle should be chosen such that the height of the tissue and blood, or microtissue and blood suspension in the receptacle does not exceed 5 mm, preferably not exceed 3 mm, or not exceed 1 mm such that tissue construct produced in the receptacle takes the shape of a layer.

The receptacle may be formed as one piece. The receptacle may also comprise two or more pieces adhered together.

The receptacle may for example comprise a plate, for example a glass plate or a thermoset polymer plate, and a frame mounted onto said plate. The frame may be a thermoplastic frame. The frame is preferably adhered, for example glued, onto the plate or with anodic bonding. Preferably the frame may be taken off, or peeled off the plate once the tissue construct has solidified in its gel-like consistency. The frame should be configured to define the surface dimension of the tissue construct.

The frame may for example be made of Polylactic acid (PLA), of poly(lactic-co-glycolic acid) (PGLA), or of thermoplastic urethane (TPU). The frame may be produced by 3D-printing.

The receptacle containing the plurality of microtissues in the blood sample is then exposed to a static acoustic wave field. For this step the receptacle is preferably placed in or on a wave generation device. The static acoustic waves cause the cells comprised in the tissue sample, respectively the microtissues to cluster into aggregates and the aggregates to arrange in a particular pattern, which is defined by the waves.

Preferably, the microtissue aggregates organise in a substantially two-dimensional pattern over the base of the receptacle. The sound energy in the static acoustic wave may also be applied to evenly distribute the microtissues across the coagulating blood layer.

Preferably individual cell or microtissue aggregates are regularly spaced with respect to the neighbouring cell or microtissue aggregates, for example at the intersection points of waves in an acoustic wave field of grid-like static waves. A regularly spaced arrangement of aggregates contributes to even growth resulting in a smooth tissue layer.

Since cells and biologically active compounds may migrate or diffuse into a tissue graft from the recipient's surrounding tissue to stimulate maturation and growth of the graft, the peripheral areas of the graft may mature more rapidly than the central areas of the graft when transplanted onto a recipient. For this reason, it may be advantageous to provide a higher density of micrograft in central portions of the graft and a lower density on the edges of the graft. An increasing density gradient of micrograft from the edges to the central portion may for example be suitable to facilitate even growth and maturation of the graft when applied to the recipient.

Aggregates of cells or microtissues may be organized in different patterns by means of the static wave field. The aggregates can be arranged in lines. The aggregates may also be organised in concentric circles. Various patterns of organising microtissue aggregates as defined by standing wave patterns are possible. The invention is not particularly limited to one specific pattern or organisation of aggregates.

The frequency of the acoustic waves is preferably comprised in a range of 10 Hz to 600 Hz, more preferably of 30 Hz to 200 Hz.

The amplitude of the acoustic waves may be set at an initial value, which may be gradually increased until the pattern of standing waves in the blood sample, also called Faraday waves or Faraday ripples, is clearly visible.

The exposure time of the sample contained in the receptacle to the static acoustic fields may vary from 1 minute to 20 minutes, preferably from 2 minutes to 10 minutes.

Based on this method, grafts for treating large wounds can be produced within a relatively short time frame. This means that the tissue grafts can be produced at bedside, in a point-of-care procedure. For example, skin grafts produced by this method can typically be ready for their application after about 40 minutes to 60 minutes production time.

The term "wound" as used herein means an injury, which damages or breaks a body tissue, for example the skin.

The very rapid provision of fully autologous tissue grafts significantly contributes to the regenerative capacity and the positive outcome of a treatment or intervention for a patient in need of the graft. Moreover, the rapid procedure reduces the risk of contamination of the graft and risk of infection of the patient in need of the graft. It also reduces the cost of surgical transplantation of tissue grafts.

Due to the high amount of micrografts which can be obtained from a tissue sample and the optimized spatial organisation of these micrografts in the sound energy application step, expansion factors of up to 40-fold, or up to 80-fold can be achieved. This expansion factor is significantly higher than the factor achieved by conventional methods, most importantly the commonly used mesh grafting, which can achieve a maximum expansion of 9-fold, but usually achieves an expansion of less than 5-fold.

A high expansion factor means that the provision of a relatively small amount of sample tissue is sufficient to generate a graft dimensioned to cover a substantial part of a surface area on a body of a recipient of the graft.

The high expansion factor achieved by this method also provides the advantage that the provision of the tissue sample by a patient has less impact on the patient, as only a relatively small amount of tissue is required. For example, with about 1 cm² of healthy skin tissue a graft covering up to 40 cm², or up to 80 cm² of surface area can be produced.

Patients in need of a large amount of tissue graft, for example due to excessive burns of large areas their body or of body parts, may not be able to provide sufficiently large areas of epithelial tissue to produce sufficiently large tissue grafts based on conventional methods. The capacity of this invention to produce large grafts based on a relatively small amount of collected tissue in a short amount of time makes a rapid provision of fully autologous tissue grafts for these patients possible.

Different from cell-based tissue engineering methods, in which grafts are produced in GMP laboratories, the present invention provides a method for point-of-care tissue production, thereby permitting for immediate and direct access to the patient to be treated.

Coagulation of the blood used for tissue construct production occurs as a natural process. A fibrin clot is formed, providing the coagulated blood's typical gel-like consistency. The coagulated blood provides a scaffold for the patterned micrografts in the receptacle.

Coagulation of the blood already occurs during the sound energy application step. Preferably, to allow the blood to coagulate, the receptacle is left without manipulation for a period of time after its sound energy application until coagulation is complete. The completion of the coagulation process can be tested mechanically, by confirming the gel-like consistency of the solidified blood layer when gently pressing against it, for example with the flat side of a spatula. In a layer of less than 5mm, the entire coagulation process is typically completed in about 30 minutes to 45 minutes.

The speed of the coagulation process is temperature-dependent. By providing lower temperatures, for example between 4°C and 25°C, coagulation is slowed down. Providing higher temperatures, for example between 35°C and 40°C the coagulation process can be accelerated.

Coagulation of the blood and microtissue mixture in the receptacle gives rise to a patterned microtissue blood sheet. The fibrin of the coagulated blood forms a provisional scaffold of the obtained tissue construct. This provisional scaffold is replaced by extracellular matrix produced when cells of the microtissue expand and/or differentiate to form a matured tissue construct.

The scaffold restricts the free movement of the aggregates within the coagulated blood tissue construct. The aggregates are immobilised in their position. During maturation of the tissue construct, for example when covering a wound of a recipient, individual cells may however migrate through the solidified tissue.

When producing tissue grafts, for example skin grafts suitable for treatment of topical wounds, the method preferably comprises a step of transferring the patterned blood tissue construct, or the tissue graft onto a non-woven support structure, for example onto a silicon support sheet, such as a wound dressing or bandage. The support structure with the tissue graft may then be suitable dimensioned to fit the required size and/or shape.

Alternatively, the sample tissue or the plurality of microtissues suspended or resuspended in the blood sample may be patterned directly on the support structure. In this embodiment the at least a part of the support structure forms a flat base of the receptacle. Preferably, in this embodiment the receptacle comprises the support structure as a base and a frame for providing the circumferential walls of the receptacle. Alternatively, the support sheet may cover the base of a receptacle.

To enable the expansion of the patterned micrografts, the patterned blood tissue construct should be incubated at ambient temperatures, or at body temperature. Preferably, the incubation may happen during the use of the tissue construct, for example, when it is applied topically to a recipient. It is however also possible, that incubation may be performed *in vitro* or *ex vivo* if the graft is not immediately applied to a patient.

To accelerate cell growth and the formation of the tissue layer, the construct may be incubated at temperatures ranging from 20°C to 40°C, preferably from 35°C to 38°C.

This invention provides a method adapted for producing 100% autologous, fully biological tissue grafts rapidly by using only a minimal amount of collected tissue. No addition of stimulants or agents, such as cytokines or growth factors, is required.

It is however possible, to add additional cytokines or growth factors to the micrograft blood suspension, to for example expedite cell growth or to stimulate cell differentiation, if desired.

The microtissue-blood construct acts as metabolically active scaffold that functions to control the release of cytokines and growth factors over several days. This controlled release facilitates the graft development and formation.

The production of more complex and/or thicker tissues constructs may be achieved by layering a series of microtissue-blood constructs or matured tissue layers.

When applied to patients, the tissue constructs stimulate the natural healing process in the wound bed and overcome the healing over the wound edges. As a result, scarring along the edges of the wound is significantly reduced. In addition, the regenerated skin covering the wound bed is not scarred and appears smoother compared to the skin produced with the common MESH split skin graft method.

By providing an autologous skin graft for a patient, the patient's natural skin colour is maintained on the site of the treated wound, as the graft contains autologous melanocytes. The graft therefore blends in better with the patients surrounding skin and is less visible.

Specific cytokines and growth factors present in the blood and/or expressed by the cells comprised in the micrograft furthermore foster angiogenesis. This contributes to a physiologically controlled regeneration of patient tissue. As a result, compared to conventional grafting methods, scarring is considerably reduced when the grafts are applied to wounds.

The invention also relates to a bank of biological tissue grafts produced by the method described herein.

In one aspect of this embodiment one or more layers may for example each comprise different types of tissue samples or microtissues.

Two or more layers may differ from each other in the type of tissue sample and/or microtissue comprised in said layers.

The layering of patterned blood tissue constructs allows for production of multi-layered tissues. Cells residing in different layers may migrate into other layers such as to create a tissue of higher complexity.

Tissue constructs, preferably single-layered constructs, generated by this invention may also be used for diagnostic purposes. The cells of the spatially dispersed microtissues in a blood construct may for example be assessed for the presence or absence of a particular biomarker or an analyte. The biomarker or the analyte may be present in or on the cells, or it may be secreted by the cells in the blood surrounding the microtissue.

Depending on the required size of the tissue construct, the process presented in this invention may be performed with relatively small quantities of blood, for example with a minimum sample volume of 300 µl, or preferably of 100 µl.

A diagnostic method using a tissue construct provided by this invention comprises the steps of exposing the tissue construct to a reagent for identifying a biological unit or an analyte of interest, detecting the presence, absence, or the level of said biological unit or analyte in the tissue construct, and comparing the obtained detection result to the expected presence, absence, or level of said biological unit or analyte in healthy tissue.

The one or more reagents may be added to the blood sample before the addition of the tissue sample or the plurality of microtissues. The one or more reagents may be added together with the tissue sample or the plurality of microtissues, or after the tissue sample or the plurality of microtissues have been suspended in the blood sample.

Alternatively or additionally, the one or more reagents may be applied to the coagulated tissue construct. In this case, the one or more reagents may be applied topically and allowed to diffuse into the tissue construct.

Based on this analysis, a diagnostic prediction regarding the presence or absence of a condition of a disease, respectively the status of a disease may be made.

Diagnostic assay as described herein may for example be performed in multi-well plates. The diagnostic assays provided by this invention are also suitable for high-through put screening, using methods known in the art.

### Short description of the drawings

Exemplar embodiments of the invention are disclosed in the description and illustrated by the drawings in which:
Figure 1 is a schematic illustration of a workflow for producing a tissue graft according to one embodiment of this invention;
Figures 2A to 2C schematically depict individual steps to transfer a tissue graft produced according to one embodiment of this invention from a receptacle to a support sheet;
Figure 3A is a prior art image showing a top view of aggregates of organoids-patterned in hydrogel over a surface following sound energy application in a static acoustic wave field;
Figure 3B is a top view image of aggregates of skin micrografts resuspended in a blood sample after mechanical disaggregation;
Figure 3C is a top view image of patterned micrografts of skin tissue in a blood sample submitted to acoustic energy in a static wave field;
Figure 4 is an image of a patterned blood tissue construct with skin micrografts placed on a silicon support sheet suitable for placements onto a wound;
Figure 5A shows images of a wound inflicted in a pig treated with a conventional MESH split skin transplant;
Figure 5B shows images of a wound inflicted in the same pig treated with a tissue graft produced according to one embodiment of this invention;
**Figure 5C** shows images of a wound inflicted in the same pig covered by wound dressing only.

### Examples of embodiments of the present invention

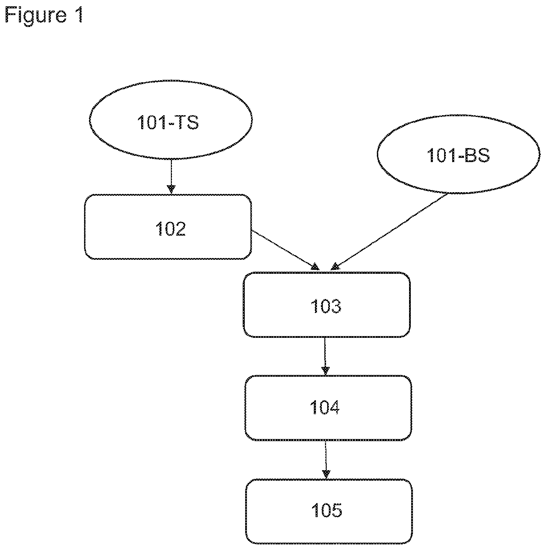

Figure 1 represents an exemplary embodiment of a workflow for the production of tissue grafts according to this invention.

In a first step 101-TS a tissue sample of the tissue needed, which was previously collected, preferably from the individual which will receive the graft, is provided. The size, respectively the amount, of the collected sample tissue depends on the dimensions of the area the graft needs to cover. This invention provides for an expansion factor of the collected tissue for more than 20-fold, depending on the tissue this may be up to about 40-fold, or up to about 80-fold.

The sample may for example have been obtained by biopsy collection. Since a relatively small amount of tissue sample is required for graft production, a punch tool is a suitable instrument to take a skin biopsy, comprising epidermal tissue and dermal tissue, for a skin tissue graft. Alternatively, dermatomes may also be used to excise a sample tissue in a procedure preceding the method of this invention. Due to the expansion factor achieved by this invention, which is significantly higher than expansion factors attained with conventional grafting methods, the trauma to the tissue donor site is kept minimal. As a result, the biopsy collection process is less painful for the donor. The site from which the tissue sample is taken is smaller and therefore heals faster. The amount of fibrosis and scarring at the smaller site is also reduced.

The tissue sample is preferably collected from the intended recipient of the produced tissue graft, in order to avoid rejection of the graft.

In a further step 102 of the procedure, the tissue sample is segregated into micrografts, preferably having a diameter of a maximum transversal dimension no more than 2 mm, than 1 mm, than 5000 µm, than 200 µm, or, preferably, of no more than 100 µm, for example 80 µm ± 20 µm.

The micrografts are preferably generated through the use of mechanical segregation methods according to procedures known in the art. A skin biopsy sample of about 1 cm² surface area can be segregated into about 6'000 to 12'000 micrografts, depending on the average size of the micrografts.

Micrografts may for example be prepared by cutting a biopsy sample into small pieces..

A blood sample collected from a donor is provided 101-BS to create a suspension of the micrografts in said blood sample 103. The donor from which the blood sample was taken in a step preceding this invention, is preferably also the intended recipient of the tissue graft. The method allows for generation of entirely autologous graft, using only blood samples and tissue samples collected from the recipient. The samples shall be freshly obtained. The samples may also be preserved samples, taken for example from a tissue bank or a blood bank.

The concentration of micrografts suspended in blood may range from 1'000 to 5'000, preferably from 2'000 to 3'000 micrografts per ml. The concentration may vary according to the average size of the micrografts and/or the amount of cells comprised in the micrografts. Tissue-specific variations are also possible.

For generation of a skin tissue graft, 7'000 to 13'000, preferably about 10'000 ± 1'000 micrografts of an average maximum transversal dimension of 80 µm are suspended in 3 ml to 6 ml, preferably about 4 ml of blood. A 4 ml suspension is suitable for the production of a skin tissue graft of about 80 cm².

The prepared micrograft suspension is provided in a receptacle having preferably a flat base member, which may be made of a thermoset polymer or a glass plate, and a frame, which defines the areal dimensions of the tissue graft. The micrograft-blood suspension should fully cover the area encompassed by the frame. The receptacle should be filled with a suitable volume of blood-micrograft suspension, such that the layer forming in the receptacle has a thickness of no more than 6 mm, or no more than 4 mm, preferably between 0.5 mm and 2 mm.

In a subsequent step 104 the receptacle containing the micrograft-blood suspension is then exposed to static acoustic waves of a frequency ranging from 10 Hz to 600 Hz, preferably from 40 Hz to 200 Hz, ideally 50Hz to 100 Hz. For this purpose, the receptacle is preferably positioned in or onto an apparatus configured to generate static acoustic wave fields. The static waves cause the micrograft to take positions predefined by the pattern of the acoustic waves. Aggregates of micrografts are preferably regularly dispersed over the base of the receptacle.

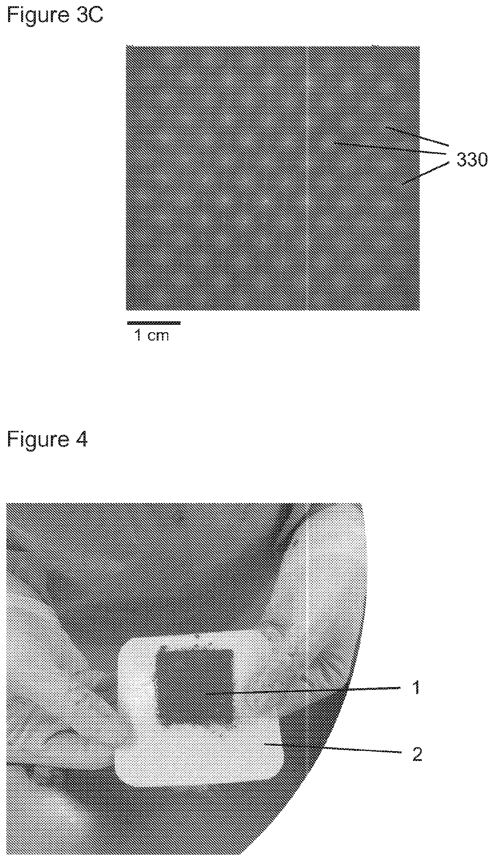

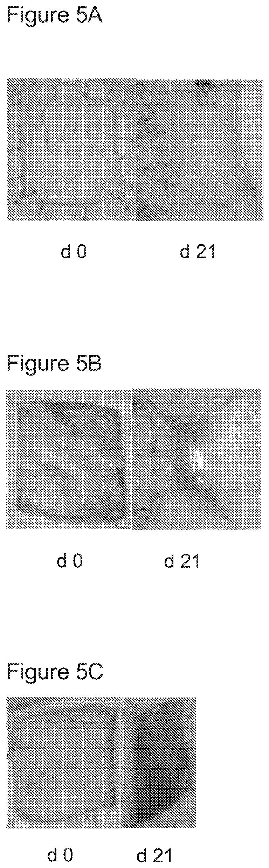

The initial amplitude of the acoustic waves may be increased gradually or stepwise increased to a maximum amplitude peak. For the generation of the skin grafts shown in Figures 4 and 5B the maximum amplitude applied was 245 milli Volt peak to peak (mVPP).

The sound energy treatment 104 may be applied for about 1 minute to 20 minutes, preferably from 2 minutes to 10 minutes. The patterned distribution of the micrograft aggregates in the receptacle may be confirmed visually.

Following the sound energy application step, the micrograft-tissue layer is preferably left unmanipulated for at least 10 minutes at ambient temperature to allow the completion of blood coagulation 105. Completion of the coagulation process typically takes 20 minutes to 40 minutes following the sound energy application step.

The clotted fibrin in the coagulated blood causes the liquid suspension to solidify into a gel-like construct. The completion of the clotting process can be confirmed mechanically, by gently pressing a flat surface against the surface of the micrograft-blood construct.

The coagulated micrograft-blood construct corresponds to a tissue construct, or tissue graft, in which the fibrin provides a provisional scaffold. Upon growth of the micrografts and maturation of the construct, the provisional scaffold is gradually replaced to give rise to a matured tissue layer.

To retrieve the skin tissue graft from the receptacle, the entire upward facing surface of the graft may be contacted with a non-woven support sheet. If the receptacle has a frame, the frame should be removed before the support sheet is applied to the micrograft-blood sheet.

A strong adherence of the tissue construct to the support sheet should be avoided, to allow for the support sheet to be removed from a transplanted tissue graft without damaging the graft.

The support sheet covers the tissue graft applied to the wound and functions as a protective barrier, protecting the wound from contact with the environment. As a result the wound is protected against infection and external mechanical impacts.

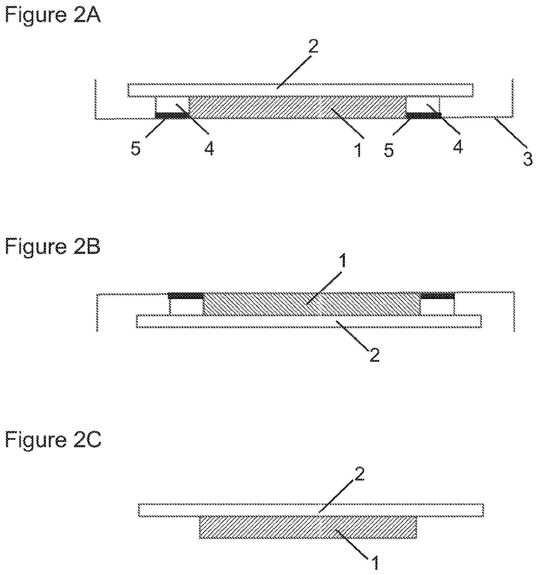

An exemplary transfer of a tissue graft from a receptacle consisting of a petri-dish and a polymer frame adhered to the base of the petri-dish is depicted in figures 2A to 2C. The figures illustrate a cross-sectional view through a receptacle containing a tissue construct.

In a first step a silicon support sheet 2 is layered onto the tissue graft 1, which is delimited by the frame 4. In this example the frame 4 is adhered to the petri-dish 3 by means of a glue 5. This "sandwich" arrangement is then turned upside down, such that the base of the petri-dish 3 faces upwards and the silicon support sheet 2 faces downwards. The petri-dish 3 and the frame 4 are then then carefully removed from the tissue graft 1. The tissue graft 1 on the support sheet 2 can then be applied to a wound of a recipient.

Alternatively, the transfer process may involve removing the frame from the base of the receptacle once the micrografts-blood construct has fully coagulated. The support sheet may then be carefully applied, preferably rolled, onto the tissue graft after removal of the frame.

The inclusion of air bubbles between the tissue graft and the support sheet should be avoided. Rolling the support sheet onto the tissue graft helps to avoid formation of such air bubbles. The receptacle base-tissue graft - support sheet assembly is then turned upside down and the receptacle base is carefully removed from the tissue graft.

Other procedures to transfer the tissue graft from the receptacle onto the support sheet are possible.

An example of a tissue graft transferred on a silicon support sheet is shown in Figure 4.

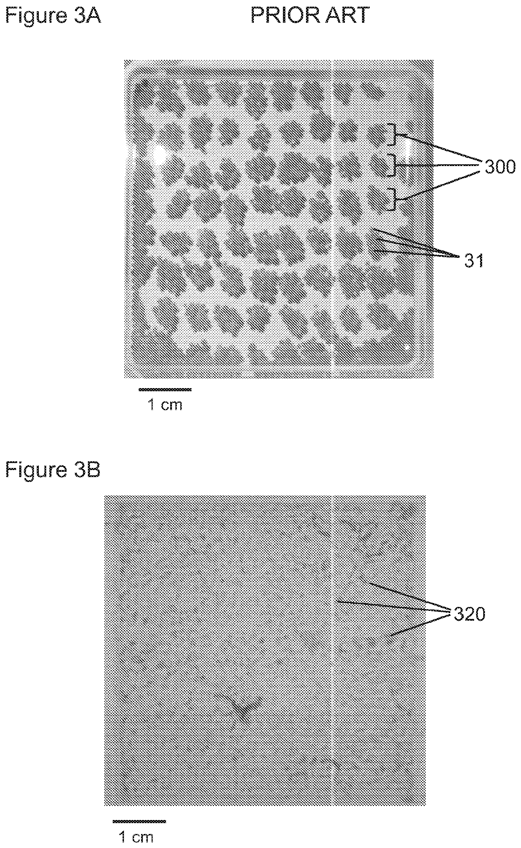

Figure 3A is a prior art image demonstrating a possible mode the patterning of aggregates upon exposure to a static acoustic wave field. The image shows aggregates 300 of organoids 31 in a variety of sizes arranged in a pattern after exposure to a static acoustic wave field as described above. The acoustic waves in the field gave caused the aggregates to form at intersection points of a substantially two-dimensional grid pattern.

The image presented in this Figure 3A is merely an example of a pattern which can be obtained for cell aggregates such as organoids by sound-induced morphogenesis.

A similar organisation of a physiological sample tissue or a plurality of microtissues can also be achieved in a sample blood as provided in this invention. The construct could be generated with microtissues, organoids, or with spheroids. A construct may also be generated with cell pellets or a suspension of cells.

The patterned aggregate-blood tissue may be incubated to let the aggregates expand and further differentiate. A physiological sample tissue, displaying the features of a particular organ may for example created in this way. The sample may also be used for diagnostic purposes as described above.

Skin grafts generated by the method disclosed in this invention were tested in a pre-clinical study on pigs. Results of the study are shown in Figures 5A to 5C.

For production of the graft, a 400 µm layer of skin tissue was removed and a blood sample was drawn from the same pig. Full thickness skin wounds of approximately 5 cm by 5 cm down to the deep fascia were inflicted on the back of the pigs. The wounds were then treated with or without tissue grafts as described below.

Skin tissue samples of 2.75 cm² and 400 µm thickness used for micrograft generation using a mechanical disaggregation method and apparatus previously described in J Cell Physiol. 2015 Oct;230(10):2299-303; doi: 10.1002/jcp.24973.

Figure 3B is an image of clustered micrografts after mechanical disaggregation of a tissue sample. The micrograft aggregates 320 shown in this image are dispersed irregularly in the blood sample.

The micrograft aggregates were concentrated in a centrifugation step and mixed with 3 ml venous blood of the donor animal and 2.5 ml were transferred into a receptacle, which was a glass plate with a frame enclosing an area of 5 cm by 5 cm. Micrograft aggregates in the blood were then exposed to Faraday standing waves at 60 Hz for approximately 2.5 minutes. Coagulation of the blood was allowed to be completed following the sound energy application process. During sound energy application and the subsequent resting period, a temperature at 35°C ± 2°C was maintained.

An image of a patterned micrograft aggregates in a blood sample is provided in Figure 3C. The patterned micrografts are not visible at the resolution of this image. The fairer portions 330 correspond to static wave valleys in the blood sample in which the micrografts are aggregated.

Once complete coagulation of the tissue graft was confirmed, the frame was removed from the glass plate and the tissue graft was transferred onto a silicone dressing having an area of 7 cm by 7 cm.

The tissue graft was then placed onto one of the wounds of the donor animal. A second wound was covered by a silicon dressing only, and a conventional meshed skin graft of the same pig was applied to a third wound. Protective dressing, including the silicon dressing, a woven gauze, a tubular stockinette and an elastic adhesive bandage, was applied to the wounds on the back of the animal.

The healing of the wounds was evaluated macroscopically, Figures 5A to 5C show images taken on day 0 and on day 21 of the treatment.

The left images in Figures 5B and 5C show open wounds before the gauze, respectively the tissue graft of this invention was applied on day 0. The left image in Figure 5A shows an open wound onto which a conventionally meshed skin graft was placed. The right images in Figures 5A, 5B and 5C show the wound after 21 days of treatment.

The wound shown in Figure 5A was treated by inserting a meshed skin graft of the same pig. The graft was not subjected to significant stretching. After 21 days the wound has healed. The inserted skin graft can easily be discerned from the surrounding skin due to its rough appearance caused by extensive scarring. The edges around the skin graft are also marked by fibrotic tissue, clearly delimiting the area of the graft from the pig's unharmed skin.

The wound shown in Figure 5B received the tissue graft produced which was prepared as described above. The healing process was not entirely completed after 21 days. However, it can already be observed that the scarring of the inserted graft is significantly less compared to the meshed skin graft. No scarring can be detected along the transition between the graft and the pig's surrounding skin. The skin looks smooth and blends in with the surrounding skin. The wound seems to heal from the outside towards the centre with the thickness of the skin in the centre being thinner compared to the peripheral areas after 21 days of treatment.

The wound shown in Figure 5C was covered by a silicon gauze and wound dressing only and did not receive any tissue graft. The wound appears somewhat reduced in size, however the wound remains substantially open and susceptible to infection.

Whilst the exemplary embodiment of producing a tissue graft, and in particular a skin graft, according to the invention has been described in detail, it will be understood by those skilled in the art, that there are multiple other embodiments of and applications for the present invention. The invention may for example be used to generate tissues, models of biological organs or to create biological units with specific biological functions. The products of this invention may for example be used for testing and experimental purposes, for diagnostic purposes or for medical therapy.

## Claims

1. Method for producing a human or animal tissue construct, comprising
- providing a tissue sample or a plurality of microtissues,
- providing a volume of a liquid sample for suspending the tissue sample,
- suspending or resuspending the tissue sample or the microtissues in said volume of liquid sample in a receptacle, said receptacle having a preferably flat base,
- exposing the receptacle containing the tissue sample or the microtissues suspended in the liquid sample to a static acoustic wave field to cause aggregates of cells comprised in the tissue sample, or aggregates of the microtissues to arrange in a pattern defined by the static acoustic waves,
**characterised in that**
- the liquid sample is a blood sample, such as a whole blood sample or a processed blood sample, wherein said processed blood sample comprises or is supplemented with coagulation promoting components for causing coagulation of the liquid sample, and
- **in that** the method comprises the step of coagulation of the blood sample to form a coagulated blood layer providing a scaffold for free migration of the aggregates, such as to create a patterned blood tissue construct.

2. The method of claim 1, further comprising an incubation step at temperatures ranging from 20°C to 40°C, ideally at 35°C to 38°C for *ex vivo* or *in vitro* to facilitate cell growth and/or maturation of the patterned blood tissue construct.

3. The method of claim 1 or 2, wherein the acoustic wave field has a frequency of 10 Hz to 600 Hz, preferably of 30 Hz to 200 Hz, or of 50 Hz to 100 Hz.

4. The method of any of claims 1 to 3, exposure time to fields ranges from 1 min to 20 mins, preferably from 2 mins to 10 mins.

5. The method of any of claims 1 to 4, wherein the microtissues are organoids, spheroids, or combinations thereof.

6. The method of any of claims 1 to 4, wherein the microtissues are micrografts.

7. The method of claim 6, wherein the micrografts have a maximum transversal dimension of no more than 1000 µm, or no more than 500 µm, preferably of no more than 100 µm, for example 80 µm ± 20 µm.

8. The method of any of claims 1 to 4, wherein the tissue sample is a suspension of cells, optionally of concentrated cells, for example derived from adipose tissue, from lymphatic tissue, from bone marrow, or from blood.

9. The method of any of claims 1 to 8, wherein multiple iterations of the method are performed to generate a series of patterned blood tissue construct which are subsequently superposed as layers.

10. The method of claim 9, wherein one or more layers each comprises different types of tissue samples or microtissues.

11. The method of claim 9 or 10, wherein two or more layers differ from each other in the type of tissue sample and/or microtissue comprised in said layers.

12. The method of any of claims 1 to 11 for generating tissue grafts, for example skin grafts, further comprising transferring the patterned blood tissue construct onto a non-woven support structure, for example onto a silicon support sheet.

13. Use of a tissue construct produced according to any of claims 1 to 11 for *in vitro* or *ex vivo* diagnostic purposes.

14. A method of diagnosis performed on a tissue construct produced according to any of claims 1 to 11, comprising the steps of
- exposing the tissue construct to a reagent for identifying a biological unit, for example a biomarker, or an analyte of interest,
- detecting the presence, absence, or the level of said biological unit or analyte in tissue construct, and
- comparing the obtained detection result to the expected presence, absence, or level of said biological unit or analyte in healthy tissue.

15. The method of claim 14 further comprising diagnosing a disease or a status of a disease based on the comparison step.

16. A bank of biological tissue grafts produced according to any of the methods 1 to 12.

## Patentansprüche

1. Ein Verfahren zur Herstellung eines menschlichen oder tierischen Gewebekonstrukts, umfassend
- Bereitstellen einer Gewebeprobe oder einer Vielzahl von Mikrogeweben,
- Bereitstellen eines Volumens einer flüssigen Probe zum Suspendieren der Gewebeprobe,
- Suspendieren oder Resuspendieren der Gewebeprobe oder der Mikrogewebe in dem Volumen der flüssigen Probe in einem Behälter, wobei der Behälter einen vorzugsweise flachen Boden aufweist,
- Aussetzen des Behälters, der die Gewebeprobe oder die in der flüssigen Probe suspendierten Mikrogewebe enthält, einem statischen akustischen Wellenfeld, um zu bewirken, dass sich Aggregate von Zellen, die in der Gewebeprobe enthalten sind, oder Aggregate der Mikrogewebe in einem durch die statischen akustischen Wellen definierten Muster anordnen,
**dadurch gekennzeichnet, dass**
- die flüssige Probe eine Blutprobe ist, wie eine Vollblutprobe oder eine verarbeitete Blutprobe, wobei die verarbeitete Blutprobe gerinnungsfördernde Komponenten enthält oder mit solchen ergänzt ist, um die Gerinnung der flüssigen Probe zu bewirken, und
- dass das Verfahren den Schritt der Koagulation der Blutprobe umfasst, um eine koagulierte Blutschicht zu bilden, die ein Gerüst für die freie Migration der Aggregate bereitstellt, um beispielsweise ein gemustertes Blutgewebekonstrukt zu erzeugen.

2. Verfahren nach Anspruch 1, das ferner einen Inkubationsschritt bei Temperaturen im Bereich von 20°C bis 40°C, idealerweise bei 35°C bis 38°C, *ex vivo* oder *in vitro* umfasst, um das Zellwachstum und/oder die Reifung des gemusterten Blutgewebekonstrukts zu erleichtern.

3. Verfahren nach Anspruch 1 oder 2, wobei das akustische Wellenfeld eine Frequenz von 10 Hz bis 600 Hz, vorzugsweise von 30 Hz bis 200 Hz, oder von 50 Hz bis 100 Hz aufweist.

4. Verfahren nach einem der Ansprüche 1 bis 3, wobei die Einwirkungszeit der Felder im Bereich von 1 Minute bis 20 Minuten, vorzugsweise von 2 Minuten bis 10 Minuten liegt.

5. Verfahren nach einem der Ansprüche 1 bis 4, wobei es sich bei den Mikrogeweben um Organoide, Sphäroide oder Kombinationen davon handelt.

6. Verfahren nach einem der Ansprüche 1 bis 4, wobei es sich bei den Mikrogeweben um Mikrografts handelt.

7. Verfahren nach Anspruch 6, wobei die Mikrotransplantate eine maximale transversale Abmessung von nicht mehr als 1000 µm, oder nicht mehr als 500 µm, vorzugsweise von nicht mehr als 100 µm, beispielsweise 80 µm ± 20 µm, aufweisen.

8. Verfahren nach einem der Ansprüche 1 bis 4, wobei es sich bei der Gewebeprobe um eine Suspension von Zellen, gegebenenfalls von konzentrierten Zellen, handelt, die beispielsweise aus Fettgewebe, Lymphgewebe, Knochenmark oder Blut stammen.

9. Verfahren nach einem der Ansprüche 1 bis 8, wobei mehrere Iterationen des Verfahrens durchgeführt werden, um eine Reihe von gemusterten Blutgewebekonstrukten zu erzeugen, die anschließend als Schichten übereinandergelegt werden.

10. Verfahren nach Anspruch 9, wobei eine oder mehrere Schichten jeweils unterschiedliche Arten von Gewebeproben oder Mikrogeweben umfassen.

11. Verfahren nach Anspruch 9 oder 10, wobei sich zwei oder mehr Schichten in der Art der Gewebeprobe und/oder des Mikrogewebes, die in diesen Schichten enthalten sind, voneinander unterscheiden.

12. Verfahren nach einem der Ansprüche 1 bis 11 zur Herstellung von Gewebetransplantaten, z. B. Hauttransplantaten, ferner umfassend das Übertragen des gemusterten Blutgewebekonstrukts auf eine vliesartigen Trägerstruktur, z. B. auf eine Silikonträgerfolie.

13. Verwendung nach einem der Ansprüche 1 bis 11 hergestellten Gewebekonstrukts für *in vitro* oder *ex vivo* diagnostische Zwecke.

14. Diagnoseverfahren, das mit einem nach einem der Ansprüche 1 bis 11 hergestellten Gewebekonstrukt durchgeführt wird, umfassend die folgenden Schritte
- Aussetzen des Gewebekonstrukts einem Reagens zur Identifizierung einer biologischen Einheit, beispielsweise eines Biomarkers, oder zur Identifizierung eines Analyten von Interesse,
- Nachweis des Vorhandenseins, der Abwesenheit oder des Gehalts der biologischen Einheit oder des Analyten im Gewebekonstrukt, und
- Vergleichen des erhaltenen Nachweisergebnisses mit dem erwarteten Vorhandensein, der Abwesenheit oder dem Gehalt der biologischen Einheit oder des Analyten in gesundem Gewebe.

15. Das Verfahren nach Anspruch 14 weiters umfassend das Diagnostizieren einer Krankheit oder eines Krankheitsstatus auf der Grundlage des Vergleichsschritts.

16. Eine Bank von biologischen Gewebetransplantaten, die nach einem der Ansprüche 1 bis 12 hergestellt wurde.

## Revendications

1. Procédé de production d'un construit tissulairehumain ou animal, comprenant
- fournir un échantillon de tissu ou une pluralité de microtissus,
- fournir un volume d'un échantillon liquide pour mettre en suspension l'échantillon de tissu,
- la mise en suspension ou la remise en suspension de l'échantillon de tissu ou des microtissus dans ledit volume d'échantillon liquide dans un récipient, ledit récipient ayant de préférence une base plate,
- l'exposition du récipient contenant l'échantillon de tissu ou les microtissus en suspension dans l'échantillon liquide à un champ d'ondes acoustiques statiques afin de provoquer l'agencement des agrégats de cellules compris dans l'échantillon de tissu, ou des agrégats de microtissus, selon un motif défini par les ondes acoustiques statiques,
**caractérisé en ce que**
- l'échantillon liquide est un échantillon de sang, tel qu'un échantillon de sang total ou un échantillon de sang traité, dans lequel ledit échantillon de sang traité comprend ou est complété par des composants favorisant la coagulation afin de provoquer la coagulation de l'échantillon liquide, et
- **en ce que** le procédé comprend l'étape de coagulation de l'échantillon de sang pour former une couche de sang coagulé fournissant un support pour la libre migration des agrégats, de manière à créer une construction de tissu sanguin structurée.

2. Procédé selon la revendication 1, comprenant en outre une étape d'incubation à des températures comprises entre 20 °C et 40 °C, idéalement entre 35 °C et 38 °C, *ex vivo* ou *in vitro,* afin de faciliter la croissance cellulaire et/ou la maturation de la structure de tissu sanguin structurée.

3. Procédé selon la revendication 1 ou 2, dans lequel le champ d'ondes acoustiques a une fréquence comprise entre 10 Hz et 600 Hz, de préférence entre 30 Hz et 200 Hz, ou entre 50 Hz et 100 Hz.

4. Procédé selon l'une des revendications 1 à 3, le temps d'exposition aux champs étant compris entre 1 minute et 20 minutes, de préférence entre 2 minutes et 10 minutes.

5. Procédé selon l'une des revendications 1 à 4, dans lequel les microtissus sont des organoïdes, des sphéroïdes ou des combinaisons de ceux-ci.

6. Procédé selon l'une des revendications 1 à 4, dans lequel les microtissus sont des microgreffons.

7. Procédé selon la revendication 6, dans lequel les microgreffons ont une dimension transversale maximale ne dépassant pas 1000 µm, ou ne dépassant pas 500 µm, de préférence ne dépassant pas 100 µm, par exemple 80 µm ± 20 µm.

8. Procédé selon l'une des revendications 1 à 4, dans lequel l'échantillon de tissu est une suspension de cellules, éventuellement de cellules concentrées, provenant par exemple de tissu adipeux, de tissu lymphatique, de moelle osseuse ou de sang.

9. Procédé selon l'une des revendications 1 à 8, dans lequel plusieurs itérations du procédé sont effectuées pour générer une série de constructions de tissus sanguins structurés qui sont ensuite superposées en couches.

10. Procédé selon la revendication 9, dans lequel une ou plusieurs couches comprennent chacune différents types d'échantillons de tissus ou de microtissus.

11. Procédé selon la revendication 9 ou 10, dans lequel deux couches ou plus diffèrent les unes des autres par le type d'échantillon de tissu et/ou de microtissu compris dans lesdites couches.

12. Procédé selon l'une des revendications 1 à 11 pour générer des greffons tissulaires, par exemple des greffons cutanés, comprenant en outre le transfert de la structure tissulaire sanguine structurée sur une structure de support non tissée, par exemple sur une feuille de support en silicone.

13. Utilisation d'une construction tissulaire produite selon l'une des revendications 1 à 11 à des fins de diagnostic *in vitro* ou *ex vivo.*

14. Procédé de diagnostic réalisé sur une construction tissulaire produite selon l'une des revendications 1 à 11, comprenant les étapes suivantes
- exposer la structure tissulaire à un réactif pour identifier une unité biologique, par exemple un biomarqueur, ou pour identifier un analyte d'intérêt,
- détecter la présence, l'absence ou le niveau de ladite unité biologique ou dudit analyte dans la structure tissulaire, et
- comparer le résultat de détection obtenu à la présence, l'absence ou le niveau attendu de ladite unité biologique ou dudit analyte dans un tissu sain.

15. La méthode selon la revendication 14 comprenant en outre le diagnostic d'une maladie ou d'un état pathologique sur la base de l'étape de comparaison.

16. Banque de greffons de tissus biologiques produits selon l'une des revendications 1 à 12.
